# EUROPÄISCHE PATENTSCHRIFT

(11) **EP 4 291 864 B1**
(45) Veröffentlichungstag und Bekanntmachung des Hinweises auf die Patenterteilung: **19.11.2025**
(21) Anmeldenummer: 22708431.6
(22) Anmeldetag: 08.02.2022
(51) Int. Cl.: G01L 19/06, G01L 9/00, A61M 1/36, A61M 5/168

(54) **VORRICHTUNG FÜR EINE EXTRAKORPORALE BLUTBEHANDLUNG**
APPARATUS FOR EXTRACORPOREAL BLOOD TREATMENT
DISPOSITIF DE TRAITEMENT SANGUIN EXTRACORPOREL

(30) Priorität: 09.02.2021 DE 102021102993
(43) Veröffentlichungstag der Anmeldung: 20.12.2023
(73) Patentinhaber: Fresenius Medical Care Deutschland GmbH, 61352 Bad Homburg (DE)
(72) Erfinder: BIERMANN, Frank, 22455 Hamburg (DE)
(74) Vertreter: Rau, Jenspeter
(86) Internationale Anmeldenummer: PCT/EP2022/052953
(87) Internationale Veröffentlichungsnummer: WO 2022/171596

(56) Entgegenhaltungen:
- EP-A1- 1 319 417
- US-A1- 2019 250 058

## Beschreibung

### GEBIET DER ERFINDUNG

Die Erfindung betrifft eine Druckmesseinrichtung und eine medizinische Funktionseinrichtung, die mit der Druckmesseinrichtung verbindbar ist. Ferner betrifft die Erfindung ein Verfahren zum Füllen der medizinischen Funktionseinrichtung, wobei die Druckmesseinrichtung zum Messen des Drucks in der gefüllten medizinische Funktionseinrichtung eingesetzt werden kann.

### HINTERGRUND

Druckmessungen in Flüssigkeiten führenden Leitungen sind beispielsweise in der Dialysetechnik erforderlich, um beispielsweise die korrekte Funktion der Dialysegeräte zu überwachen.

Bei der Hämodialyse, bei dem Blut extrakorporal gereinigt wird mittels eines Dialysegeräts, als ein Beispiel für eine extrakorporale Blutbehandlungsvorrichtung, können daher an verschiedenen Stellen des Blut führenden Systems eine oder mehrere Druckmesseinrichtungen vorgesehen sein. Diese sind beispielsweise:
- zwischen einem patientenseitigen Ende einer arteriellen Leitung und einer Pumpe, die entlang dieser arteriellen Leitung angeordnet sein kann (arterielle Druckmesseinrichtung),
- zwischen der Pumpe und einem Dialysator (Vorfilter-Druckmesseinrichtung),
- an einer venösen Leitung, mittels der das Blut vom Dialysator in den Patienten zurückgeführt wird, zwischen dem Dialysator und einer Luftabscheidekammer (Nachfilter-Druckmesseinrichtung).

Als Druckmesseinrichtungen sind dabei verschiedene Drucksensoren bekannt. So werden beispielsweise Drucksensoren eingesetzt, bei denen der Druck über eine luft- und flüssigkeitsundurchlässige Membran anhand der Auslenkung der Membran detektiert wird. Andere Drucksensoren sehen ein Luftpolster vor, das derart positioniert ist, dass die Luft in dem Luftpolster bei einem höheren Druck in der Flüssigkeit führenden Leitung, komprimiert wird und mittels eines mit diesem Luftpolster verbundenen Drucksensors der Druck gemessen werden kann. Nachteil dieser letzten Lösung ist insbesondere, dass es hier während der Behandlung eine Blut-Luft-Grenzfläche gibt. Solche Druckmesseinheiten mit einem Luftpolster weisen zum Schutz der maschinenseitigen Luftleitungen oft ein Druckübertragungselement auf. Dieser weist eine im trockenen Zustand luftdurchlässige hydrophobe Membran auf, die für Flüssigkeit undurchlässig ist. Damit die Druckmesseinheit funktioniert, darf diese hydrophobe Membran nicht befeuchtet werden, da dann die Luft bei einer Veränderung des Drucks in der Flüssigkeit führenden Leitung nicht durch die Membran hindurchtreten kann und dadurch die Luft in dem Luftpolster nicht mehr komprimiert werden kann.

Um jedoch den Druck in einer Flüssigkeit führenden Leitung messen zu können, muss die Leitung zunächst gefüllt werden. Mit anderen Worten, die Luft in der Leitung muss durch die Flüssigkeit ersetzt werden. Dieser Vorgang wird in der Dialyse Primen oder Füllen genannt, wobei in diesem Schritt der Patient noch nicht mit dem Dialysegerät, insbesonders nicht mit der arteriellen oder der venösen Leitung, verbunden ist. Ein möglichst vollständiges Entfernen der Luft ist aus den Leitungen ist deshalb wichtig, weil sonst dem Patienten Luft in die Vene zugeführt werden könnte.

Für diesen Füllvorgang gibt es viele Lösungen. So kann die Luft über eine Druckmesseinheit mit Luftpolster wie sie oben beschrieben ist, die zusätzlich über eine Öffnung nach außen aufweist, abgeschieden werden. Mit anderen Worten, während des Primings/Füllens wird die Luft über die hydrophobe Membran in Richtung des Luftdrucksensors verschoben und nach außen abgeleitet. Während dieses Vorgangs ist das Druckmesssystem nach außen offen.

EP1319417 beschreibt ein Verfahren zur Überwachung eines an ein medizinisches Instrument angeschlossenen Fluidleitungssystems. Dieses Verfahren kann unter Nutzung einer von einem oberen Ende einer Tropfkammer abzweigenden Sensorleitung, in der ein eine hydrophobe Membran aufweisender Gassensorprotektor und an deren Ende ein Gasdrucksensor angeordnet sind, erfolgen.

US2019250058 beschreibt eine Druckmessvorrichtung mit einem rohrförmigen Druckanschluss, der eine Innenabschrägung angrenzend an eine auslenkbare Membran und/oder eine hydrophobe oder superhydrophobe Beschichtung aufweist, die auf mindestens einen Teil des rohrförmigen Druckanschlusses, der Innenabschrägung und/oder der auslenkbaren Membran aufgebracht ist.

Aufgabe der Erfindung ist es, eine neuartige Druckmesseinrichtung anzugeben, die die verschiedenen Funktionen herkömmlicher Druckmesseinrichtungen erfüllt, ohne die Nachteile dieser mit sich zu bringen.

### BESCHREIBUNG DER ERFINDUNG

Eine Druckmesseinrichtung, wie in Anspruch 1 definiert, weist eine hydrophobe Membran, die im trockenen Zustand luftdurchlässig ist und in einem befeuchteten Zustand luftundurchlässig ist, und einen Drucksensor, der mit der hydrophoben Membran in mechanischen Kontakt steht und der eingerichtet ist, einer Bewegung der Membran zu folgen, auf.

Die Druckmesseinrichtung weist ferner eine erste Kammer, die auf einer Vorderseite der hydrophoben Membran angeordnet ist, und eine zweite Kammer, die auf einer Rückseite der hydrophoben Membran angeordnet ist, auf.

Der Drucksensor in der zweiten Kammer ist auf der hydrophoben Membran angeordnet.

Der Drucksensor der Druckmesseinrichtung kann eine Druckmesssensorik, insbesondere eine Dehnungsmessstreifensensorik oder einen piezoelektrischen Sensor, aufweisen. Der Drucksensor kann ferner eine Trägerschicht, auf dem die Druckmesssensorik angeordnet ist, aufweisen. Die Druckmesssensorik kann auf der der hydrophoben Membran abgewandten Seite der Trägerschicht angeordnet sein.

Die Trägerschicht kann eine elastische Membran aufweisen. Der Randbereich der Trägerschicht kann an der ersten Kammer oder der zweiten Kammer vorgespannt befestigt sein. Die Trägerschicht kann auf der hydrophoben Membran befestigt ist.

Die erste Kammer der Druckmesseinrichtung kann gemeinsam mit der hydrophoben Membran lösbar an der zweiten Kammer befestigt sein.

Die erste Kammer der Druckmesseinrichtung kann gemeinsam mit der hydrophoben Membran und dem Drucksensor lösbar an der zweiten Kammer befestigt sein.

Der Drucksensor der Druckmesseinrichtung kann ferner eine Kabelleitung aufweisen, um ein elektrisches Signal an eine Auswerteeinheit zu übermitteln, wobei die Kabelleitung optional eine Konnektoreinheit aufweisen kann zum Verbinden und/oder Lösen des Drucksensors mit der Auswerteeinheit.

Die erste Kammer der Druckmesseinrichtung kann wenigstens zwei Kanäle aufweisen, die jeder an einem Ende von der hydrophoben Membran abgeschlossen werden. Die wenigstens zwei Kanäle können einzeln absperrbar sind.

Eine medizinische Funktionseinrichtung kann wenigstens eine Blutleitung und/oder einen Dialysator aufweisen zur Verwendung mit der Druckmesseinrichtung, wobei die erste Kammer und die hydrophobe Membran Teil der medizinischen Funktionseinrichtung sind. Alternativ kann die erste Kammer und die hydrophobe Membran und der Drucksensor Teil der medizinischen Funktionseinrichtung sein.

Die medizinische Funktionseinrichtung kann einen Leitungsabschnitt aufweisen, der zum Einlegen in eine peristaltische Pumpe eingerichtet ist. Die medizinische Funktionseinrichtung kann einen Impeller zur Verwendung in einer Impellerpumpe aufweisen. Die medizinische Funktionseinrichtung kann eine Kammer mit einer Membran zur Verwendung in einer Membranpumpe aufweisen. Die Druckmesserinrichtung kann fluidisch vor oder hinter der Pumpe angeordnet sein. Die medizinische Funktionseinrichtung kann eine Luftabscheidekammer aufweisen, wobei die Druckmesseinrichtung fluidisch vor oder hinter der Luftabscheidekammer angeordnet sein kann. Die medizinische Funktionseinrichtung kann einen Dialysator aufweisen, wobei die Druckmesseinrichtung an dem Dialysator angeordnet sein kann.

Ein Verfahren zum Messen eines Drucks unter Verwendung der Druckmesseinrichtung weist folgende Schritte auf:
- Füllen des Blutschlauchsystems mit einer Flüssigkeit unter Verdrängung von Luft im Blutschlauchsystem durch die hydrophobe Membran hindurch, wobei die hydrophobe Membran von er Flüssigkeit benetzt wird, so dass sie luftundurchlässig wird oder im Bereich von einem der wenigstens zwei Kanäle luftundurchlässig wird, und
- Messen der Veränderung der Lage der Membran, insbesondere seiner Form, durch den Drucksensor.

Der Begriff "Druckmesseinrichtung" im Sinne dieser Beschreibung hat die Bedeutung, dass zumindest wesentliche erfindungsgemäße Elemente zum Messen von Druck umfasst sind. Der Begriff ist nicht so zu verstehen, dass sämtliche Elemente, die technisch erforderlich sind, um eine Druckmessung beispielsweise auch auszuwerten, davon umfasst sein müssen. Beispielweise, kann der Drucksensor vorsehen, eine veränderte Wölbung der Membran zu messen, eine Auswerteeinheit, die diese Wölbung in einen Druckwert übersetzt, kann in dem Drucksensor selbst integriert sein, kann aber auch als separate Einheit bereitgestellt werden. In diesem Fall muss die Auswerteeinheit nicht zwingend Teil der Druckmesseinrichtung sein.

Die Figuren zeigen:
Fig. 1 zeigt schematisch einen Aufbau einer Druckmesseinrichtung;
Fig. 2 zeigt schematisch eine Ausführungsform des Aufbaus eines Drucksensors;
Fig. 3 zeigt schematisch einen Schichtaufbau der Druckmesseinrichtung;
Fig. 4a bis 4c zeigen schematisch Ausführungsformen einer Trägerschicht;
Fig. 5 zeigt schematisch einen Aufbau einer Druckmesseinrichtung mit einer ersten und einer zweiten Kammer;
Fig. 6 und 7 zeigen schematisch wie die Druckmesseinrichtung in einen Einwegartikel und einen Behandlungsvorrichtung integriert sein kann;
Fig. 8a und 8b zeigen schematisch Ausführungsformen der mechanischen Kopplung eines Drucksensors mit einer hydrophoben Membran;
Fig. 9 zeigt schematisch eine Ausführungsform der Kopplung eines Drucksensors mit einer hydrophoben Membran unter Verwendung eines Unterdrucks;
Fig. 10 zeigt schematisch eine Ausführungsform eines Behandlungssystems mit einer Druckmesseinrichtung;
Fig. 11 zeigt schematisch eine Ausführungsform einer Druckmesseinrichtung, bei der die Druckmesseinrichtung in einer Kappe eines Dialysators integriert ist;
Fig. 12 zeigt schematisch eine Ausführungsform einer Druckmesseinrichtung mit mehreren Kanälen als erste Kammer;
Fig. 13 zeigt schematisch eine Ausführungsform einer Belüftungseinrichtung;
Fig. 14 zeigt schematisch eine Ausführungsform der Kanäle in Form flexibler Elemente und der Verschlussmittel einer Belüftungseinrichtung;
Fig. 15 zeigt schematisch eine Ausführungsform der Kanäle und des Verschlussmittels in Form eines Schiebers einer Belüftungseinrichtung; und
Fig. 16 zeigt schematisch die Integration einer Belüftungseinrichtung in eine Kappe eines Dialysators.

Bei der Beschreibung der Ausführungsformen unter Verweis auf die Figuren werden gleich oder vergleichbare Merkmale mit denselben Referenzzeichen gekennzeichnet. Solche gleichen oder vergleichbaren Merkmale werden nicht bei jeder Figur erneut beschrieben, dazu wird auf die jeweilige Beschreibungsabschnitte, in denen diese Merkmale zuvor bereits beschrieben wurden, verwiesen.

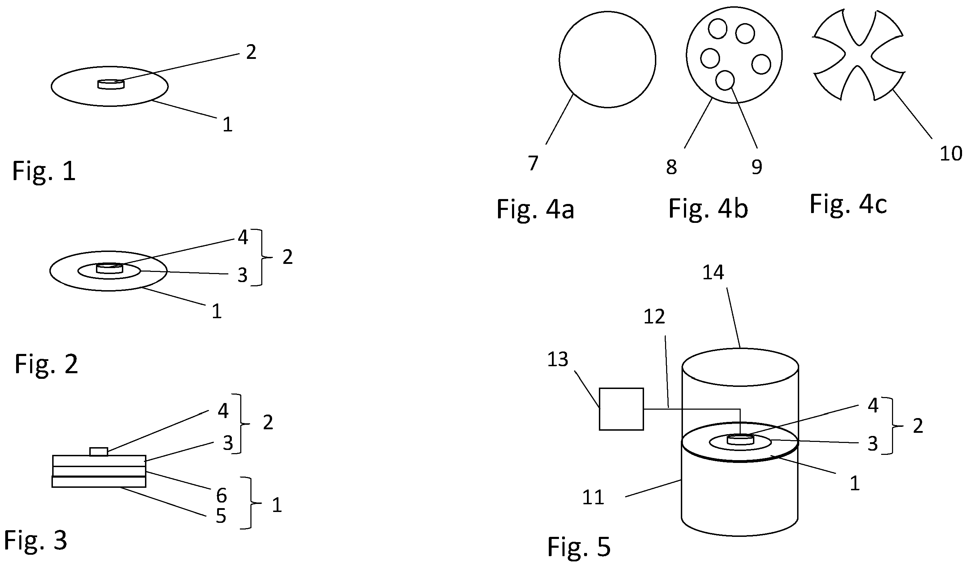

Fig. 1 zeigt einen schematischen Aufbau einer Druckmesseinrichtung. Die Druckmesseinrichtung weist eine hydrophoben Membran 1 auf und einen Drucksensor 2. Die hydrophobe Membran 1 ist im trockenen Zustand luftdurchlässig und ist in einem befeuchteten Zustand luftundurchlässig. Die hydrophobe Membran 1 ist auch undurchlässige für wasserbasierte Flüssigkeiten. Bei diesen Flüssigkeiten kann es sich um Wasser, Primingflüssigkeit (Füllflüssigkeit), Dialysat oder Blut handeln. Der Drucksensor 2 steht mit der hydrophoben Membran 1 in mechanischen Kontakt. Durch diesen mechanischen Kontakt kann der Drucksensor 2 eine Bewegung der Membran 1 detektieren. Dabei handelt es sich beispielsweise um eine Bewegung einer am Rand befestigten hydrophoben Membran 1, die zu einer Wölbung der hydrophoben Membran 1 führt. Die Bewegung kann auch eine translatorische Bewegung der hydrophoben Membran 1. Die Bewegung der hydrophoben Membran 1 kann beispielsweise durch eine Druckänderung auf der dem Sensor abgewandten Seite (Vorderseite) der hydrophoben Membran 1 bewirkt werden.

Der Drucksensor 2 kann, wie in Fig. 2 schematisch dargestellt, eine Trägerschicht 3 aufweisen. Auf der Trägerschicht 3 kann die Druckmesssensorik 4 des Drucksensors 2 angeordnet sein. Die Trägerschicht 3 kann zwischen Druckmesssensorik 4 und der hydrophoben Membran 1 angeordnet sein. Mit anderen Worten, die Druckmesssensorik 4 kann auf der der hydrophoben Membran abgewandten Seite (Rückseite) der Trägerschicht 3 angeordnet sein. Die Bewegung der hydrophoben Membran 1 kann über die Trägerschicht 3 auf die Druckmesssensorik 4 übertragbar sein. Die Druckmesssensorik 4 der Drucksensors 2 kann eine Dehnungsmessstreifensensorik oder einen piezoelektrischen Sensor aufweisen.

Der Ausdruck Druckmesseinrichtung beschreibt eine Einrichtung, mit der ein Druck ermittelt werden kann. Die Druckmesseinrichtung kann einen Drucksensor aufweisen. Der Ausdruck Drucksensor beschreibt eine Einrichtung, die auf eine Veränderung des Drucks reagiert. Der Drucksensor kann eine Trägerschicht und eine Druckmesssensorik aufweisen, wobei der Ausdruck Druckmesssensorik eine Einrichtung beschreibt, die bei Veränderung des Drucks eine Veränderung erfährt und dabei ein Signal erzeugen kann.

Die Trägerschicht 3 kann fest oder lösbar mit der Druckmesssensorik 4 verbunden sein.

Die Trägerschicht 3 kann fest oder lösbar mit der hydrophoben Membran 1 verbunden sein.

Die hydrophobe Membran 1 kann, wie in Fig. 3 schematisch dargestellt, wenigstens eine Schicht 5 eines hydrophoben Materials aufweisen oder daraus bestehen. Das hydrophobe Material kann einen hydrophoben Kunststoff aufweisen oder daraus bestehen. Beispielsweise kann der hydrophobe Kunststoff PTFE (Polytetrafluorethylen) oder ePTFE (expanded Polytetrafluoroethylen) sein oder aufweisen. Der hydrophobe Kunststoff kann auch ein Polyolefin oder ein Silicon sein oder aufweisen.

Die hydrophobe Membran 1 kann aus zwei oder mehr Schichten bestehen oder diese aufweisen, beispielsweise eine erste Schicht 5 aus dem hydrophoben Material oder dieses aufweisend und einer zweite Schicht 6, die eine Drainage- und/oder Stützfunktion hat und aus Gewebe und/oder Vlies besteht oder dieses aufweist, das schweißbar und/oder klebbar ist. Die hydrophobe Membran 1 kann alternativ neben den zuvor genannten Schichten weitere Schichten oder Bestandteile aufweisen. Die erste Schicht 5 kann mit der zweiten Schicht 6 flächig oder lediglich im Randbereich entlang des Umfangs der ersten Schicht 5 und der zweiten Schicht 6 verbunden, beispielsweise verschweißt oder verklebt, sein.

Die Trägerschicht 3 kann auch die zweite Schicht 6 der hydrophoben Membran 1 sein oder die zweite Schicht 6 der hydrophoben Membran 1 kann eine Schicht sein, die zusätzlich zur Trägerschicht 3 vorliegt.

Die Trägerschicht 3 kann eine Kunststoffschicht sein. Bei der Trägerschicht 3 kann es sich um eine dünne flexible Keramikschicht handeln oder diese aufweisen. Die Trägerschicht 3 hat eine Flexibilität, so dass sie Bewegungen der hydrophoben Membran 1 übertragen kann.

Mit anderen Worten kann der Schichtaufbau wie folgt sein:
Auf der Vorderseite eine hydrophobe Membranschicht 5, darauf angeordnet optional eine Schicht 6 mit Drainage- und/oder Stützfunktion, darauf angeordnet optional die Trägerschicht 3, darauf angeordnet die Druckmesssensorik 4.

In den Figuren 4a bis 4c sind Ausführungsformen der Trägerschicht 3 schematisch dargestellt. Die Trägerschicht 3 kann eine geschlossene Membran 7 aufweisen. Der Begriff "geschlossen" hat hier die Bedeutung, dass die Membran keine makroskopischen Öffnungen aufweist, sondern ein über die Fläche homogenes Material aufweist Die geschlossene Membran 7 kann die gesamte Fläche der hydrophoben Membran 1 bedecken. Da in dieser Ausführungsform der geschlossenen Membran 7 die Luft, die durch die hydrophobe Membran 1 hindurchtritt, auch durch die geschlossene Membran 7 hindurchtreten muss, ist die geschlossenen Membran 7 luftdurchlässig. Die geschlossene Membran 7 kann in einer weiteren Ausführungsform die hydrophobe Membran nur teilweise bedecken. In einer solchen Ausführungsform kann die geschlossene Membran 7 auch luftundurchlässig sein. Die Trägerschicht 3 kann auch eine geöffnete Membran 8 aufweisen oder daraus bestehen. Der Begriff "geöffnet" hat hier die Bedeutung, dass die Membran eine oder mehrere makroskopischen Öffnungen 9 aufweist. Die Fläche der Öffnungen 9 können in der Größenordnung von 1 bis 1000 Quadratmillimetern oder in der Größenordnung von 1 bis 10 Quadratzentimetern liegen. Die geöffnete Membran 8 kann in einer Ausführungsform einen geschlossenen Randbereich aufweisen oder in einer weiteren Ausführungsform können die Öffnung auch den Randbereich umfassen, wie schematisch in Fig. 4c gezeigt, so dass die die geöffnete Membran 8 eine Stegmembran 10 ist.

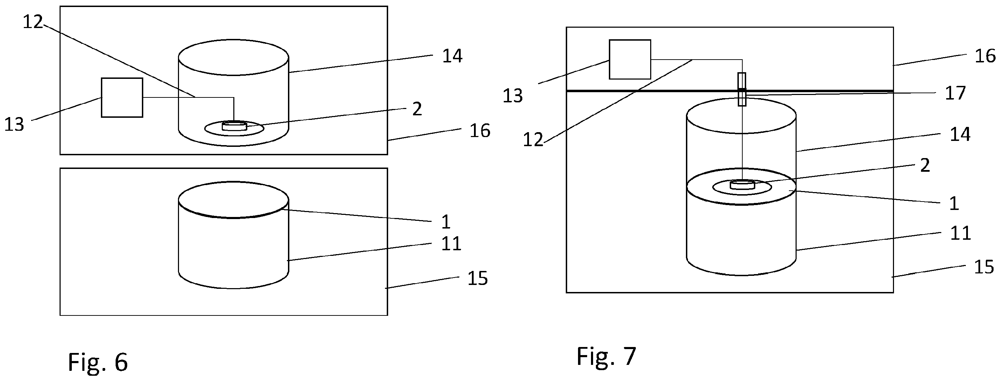

Die Druckmesseinrichtung kann, wie in Fig. 5 schematisch dargestellt, eine erste Kammer 11 aufweisen. Ein Teil der Wandung der ersten Kammer 11 kann durch die hydrophobe Membran 1 abgeschlossen werden. Die hydrophobe Membran oder, soweit vorhanden die zweite Schicht 6, kann an ihrem äußeren Rand fluiddicht mit der ersten Kammer 11 verbunden sein. Die Verbindung kann ein Verkleben, Verschweißen, und/oder Bördeln sein. Die erste Kammer 11 kann aus einem Hartplastik beispielsweise Polypropylen oder Hart-PVC gebildet sein oder die Wandung kann dieses Material aufweisen. Die erste Kammer 11 kann eine Durchflusskammer sein. Die Durchflusskammer kann in Ihrer Wandung zwei oder mehr Öffnungen aufweisen. Die erste Kammer 11 kann eine Sackgassenkammer sein. Die Sackgassenkammer kann genau eine Öffnung aufweisen. Die erste Kammer 11 kann Teil eines Einwegartikels (Disposable) sein. Bei dem Einwegartikel kann es sich um ein Schlauchset, eine Kassette oder einen Dialysator handeln. Bei dem Einwegartikel kann es sich insbesondere um ein Fluidleitungssystem handeln, das für eine Dialysebehandlung, beispielsweise einer Hämodialyse, Hämofiltration, Hämodiafiltration, Ultrafiltration, Zwerchfelldialyse, Bauchfelldialyse vorgesehen ist. Der Begriff Einwegartikel hat dabei die Bedeutung, dass der Einwegartikel für eine Behandlung eines Patienten an dem Behandlungsvorrichtung vom Nutzer angebracht wird, und zwar in der Regel für jede Behandlung erneut ein neuer Einwegartikel. Normalweise sollen aus hygienischem Gründen Einwegartikel lediglich einmal verwendet werden, jedoch können beispielsweise Kostenaspekte dazu führen, dass Einwegartikel mehrfach verwendet werden. Entscheidend ist, dass ein Einwegartikel nicht dauerhafter Bestandteil der Behandlungsvorrichtung ist, sondern lediglich während der Behandlung eine Funktionseinheit mit der Behandlungsvorrichtung bildet. Dazu wird auch auf die Beschreibung zu den Figuren 6 und 7 verwiesen.

Die Druckmesssensorik 4 kann über eine Signalleitung 12 mit einem Prozessor 13 verbunden sein. Der Prozessor 13 kann programmiert sein, ein von der Druckmesssensorik 4 übertragenes Signal zu verarbeiten und daraus einen Druckwert zu ermitteln. Der Prozessor 13 kann diesen Druckwert an einen weiteren Prozessor weitergeben, wobei der weitere Prozessor programmiert sein kann, auf der Grundlage des Druckwertes einen oder mehrere Verfahrensschritte durchzuführen oder diese zu veranlassen. Der Prozessor 13 kann programmiert sein, auch selbst die Funktionen des weiteren Prozessors auszuführen. Der Prozessor 13 kann Teil einer Steuervorrichtung sein. Die Steuereinrichtung kann den Prozessor 13 und optional den weiteren Prozessor, einen Speicher und einen Kommunikationsbus aufweisen. Der Begriff Prozessor ist dabei nicht auf einen einzelnen physischen Prozessor beschränkt. Vielmehr kann der Prozessor auch ein Multikernprozessor oder andersartig miteinander verbundene Prozessoren oder Prozessorbestandteile aufweisen. Der Speicher kann ein volatiler und/oder nichtvolatiler Speicher oder eine Kombination derselben sein. Der Datenbus kann zum Austausch von Daten zwischen Speicher und Prozessor dienen. Die Steuervorrichtung kann die dem Fachmann bekannten physischen Bausteine eines Computers aufweisen, die erforderlich sind, ein Programm ablaufen zu lassen und damit eine medizinische Vorrichtung anzusteuern. Zu solchen Bausteinen können neben den oben genannten Prozessoren, Speicher und Kommunikationsbus auch Schnittstellen zu den anzusteuernden Vorrichtungen, wie Aktoren, Sensoren, Anzeige, drahtgebundene und drahtlose Kommunikationen und ähnliches zählen.

Die Druckmesseinrichtung weist, wie in Fig. 4 schematisch dargestellt, eine zweite Kammer 14 auf. auf der Rückseite der hydrophoben Membran 1 angeordnet.

Die zweite Kammer 14 kann von der hydrophoben Membran 1 und/oder der zweiten Schicht 6 und/oder der Trägerschicht 3 zumindest teilweise abgeschlossen werden. Die Druckmesssensorik ist in der zweiten Kammer 14 angeordnet.

Die zweite Kammer 14 kann Teil der Behandlungsvorrichtung oder des Einwegartikels sein. Die zweite Kammer 14 kann aus demselben Material wie die erste Kammer 11 oder einem anderen Material bestehen. Insbesondere kann die zweite Kammer 14 als Teil der Behandlungsvorrichtung aus Metall bestehen oder dieses aufweisen.

Die Figuren 6 und 7 zeigen schematisch wie die Druckmesseinrichtung in einen Einwegartikel 15 und eine Behandlungsvorrichtung 16 integriert sein kann. Die Druckmesseinrichtung kann derart aufgebaut sein, dass die hydrophobe Membran 1 von dem Drucksensor 2 trennbar ausgebildet ist. Die hydrophobe Membran 1 und optional die erste Kammer 11 kann Teil des Einwegartikels 15 sein und der Drucksensor 2 und optional die zweite Kammer 14 kann Teil der Behandlungsvorrichtung 16 sein. Die Behandlungsvorrichtung 16 kann die Signalleitung 12 und den Prozessor 13 aufweisen. Bei Gebrauch der Druckmesseinrichtung werden der Drucksensor 2 und die hydrophobe Membran 1 miteinander mechanisch gekoppelt, so dass sich eine Bewegung der hydrophoben Membran 1 auf den Drucksensor 2 überträgt. In einer alternativen Ausführungsform sind die hydrophobe Membran 1 und optional die erste Kammer 11, der Drucksensor 2 und optional die zweite Kammer 14 Teil des Einwegartikels. Die Signalleitung 12 ist trennbar ausgebildet und weist einen Konnektor 17 auf. Der Konnektor 17 weist einen ersten Anschluss auf der Seite des Einwegartikels auf und einen zweiten Anschluss auf der Seite der Behandlungsvorrichtung 16 auf. Die Behandlungsvorrichtung 16 kann zumindest einen Teil der Signalleitung 12 und den Prozessor 13 aufweisen. Bei Gebrauch der Druckmesseinrichtung werden der erste Anschluss und der zweite Anschluss miteinander verbunden.

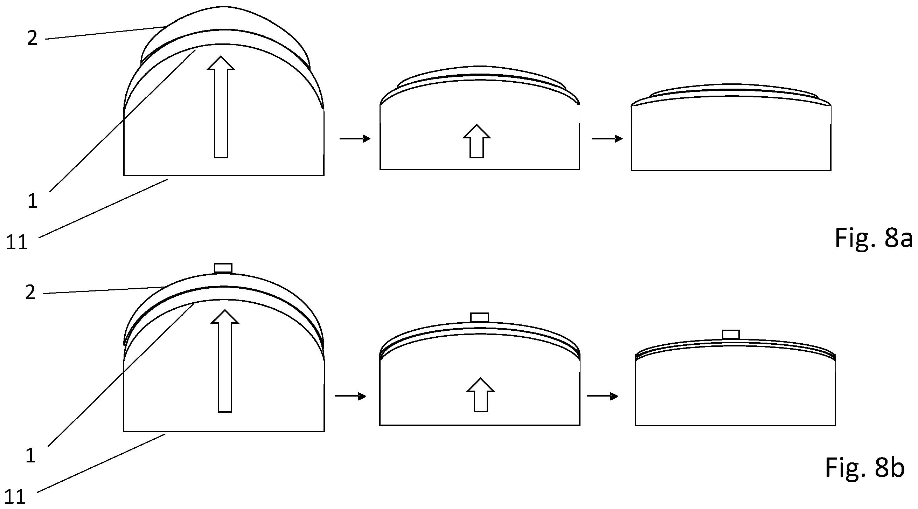

Die Kopplung der Bewegung der hydrophoben Membran 1 mit dem Drucksensor 2 kann auf verschiedene Arten erfolgen. Bei dieser Bewegung ist die hydrophobe Membran 1 nicht luftdurchlässig, also in einem benetzten Zustand. Dadurch kann ein Druckausgleich über die hydrophobe Membran verhindert werden bzw. blockiert sein. Diese wird unter Bezugnahme auf die Figuren 8a und 8b beschrieben. Druck bedeutet dabei den Differenzdruck über die hydrophobe Membran 1.

Die hydrophobe Membran 1 oder die oben beschriebene zweiten Schicht weisen eine Elastizität auf. Durch die Elastizität folgt die hydrophobe Membran 1 oder die oben beschriebene zweiten Schicht dem Druck. Elastisch bedeutet dabei, dass die hydrophobe Membran 1 oder die oben beschriebene zweite Schicht derart ausgebildet ist, dass eine Rückstellkraft auf das Material wirkt, so dass sie ohne Druckeinwirkung eine flachere Konfiguration aufweist als unter Druckbelastung. Der Begriff "flach" bzw. "flacher" bedeutet dabei eine geringere Auswölbung. Die hydrophobe Membran 1 oder die oben beschriebene zweite Schicht kann vorgespannt sein. "Vorgespannt" bedeutet dabei eine Befestigung der hydrophoben Membran 1 oder der oben beschriebenen zweiten Schicht an einer Wandung, so dass bei einem Lösen der Befestigung von der Wandung die hydrophobe Membran 1 oder die oben beschriebenen zweiten Schicht kontrahiert.

Der Drucksensor 2 kann unmittelbar mit der hydrophoben Membran 1 oder der oben beschriebenen zweiten Schicht, die mit der hydrophoben Membran 1 verbunden ist, verbunden sein. Die hydrophobe Membran 1 und/oder die zweite Schicht kann fest mit einer Wandung der ersten Kammer 11 verbunden sein. Wird nun ein großer Druck angelegt (angedeutet durch den langen Pfeil in Fig. 8a und 8b), wird die hydrophobe Membran und soweit vorhanden die zweite Schicht ausgewölbt und damit gestreckt. Damit wird der Drucksensor 2 ebenfalls gestreckt. Wird der Druck verringert (kürzerer Pfeil) bzw. auf null herabgesetzt (kein Pfeil), verkleinert sich die Auswölbung und damit die Streckung des zweiten Sensors.

Eine weitere Ausführungsform, schematisch dargestellt in Figur 8b, unterscheidet sich von der im Zusammenhang mit Figur 8a beschriebenen Ausführungsform dadurch, dass bei dieser Ausführungsform der Drucksensor 2 selbst an der Wandung der ersten Kammer 11 oder der zweiten Kammer befestigt ist. Insbesondere kann die Befestigung eine Befestigung der Trägerschicht des Drucksensors 2 sein. Der Drucksensor 2 kann elastisch ausgestaltet sein und/oder vorgespannt befestigt sein. Zur Erklärung dieser Merkmale wird auf die Erklärung zur hydrophoben Membran 1 verwiesen. In dieser Ausführungsform kann der Drucksensor 2 nicht mit der hydrophoben Membran 1 und nicht mit der oben beschriebenen zweiten Schicht verbunden sein. Dabei können die hydrophobe Membran 1 und die oben beschriebene zweite Schicht keine Vorspannung aufweisen. Wird bei dieser Ausführungsform ein großer Druck angelegt, wird die luftundurchlässige bzw. befeuchtete hydrophobe Membran 1 gegen den Drucksensor 2 gedrückt, dieser wölbt sich aus und erlaubt damit eine Druckmessung. Bei Verringerung des Drucks zieht sich der Drucksensor 2 zusammen und die Auswölbung verringert sich, bis bei einem Druck von 0 der Drucksensor 2 vollständig in Richtung der Auswölbung relaxiert ist. Diese Ausführungsform ist lediglich geeignet zum Messen von positiven Drücken, da bei negativen Drücken die hydrophobe Membran 1 weiter entgegen der Pfeilrichtung in Figur 8 gezogen wird, da aber keine feste Kopplung zwischen der hydrophoben Membran 1 und dem Drucksensor 2 vorliegt, folgt der Drucksensor 2 der hydrophoben Membran 1 nicht. Eine solche Ausführungsform kann besonders dort gut eingesetzt werden, wo lediglich positive Druckwerte gemessen werden sollen. Ein Vorteil dieser Ausführungsform ist, dass es sich dabei um einen relativ einfachen und kostengünstigen Aufbau handelt und vor allem auf die Kopplung verzichtet werden kann. Es hat sich gezeigt, dass die Kopplung ansonsten zum Beispiel während einer Behandlung geprüft werden sollte, was einen entsprechenden gerätetechnischen und/oder verfahrenstechnischen und/oder zeitlichen Aufwand bedeutet. Diese Ausführungsform kann beispielsweise in Kombination mit der im Zusammenhang mit Fig. 6 beschriebenen Ausführungsform verwirklicht sein. Bei dieser sich ergebenden Ausführungsform, muss lediglich der vorgespannte, behandlungsseitige Drucksensor bei Einbau des die hydrophobe Membran aufweisenden Einmalartikel mit dieser Membran in Kontakt gebracht werden und die positiven Drücke können gemessen werden.

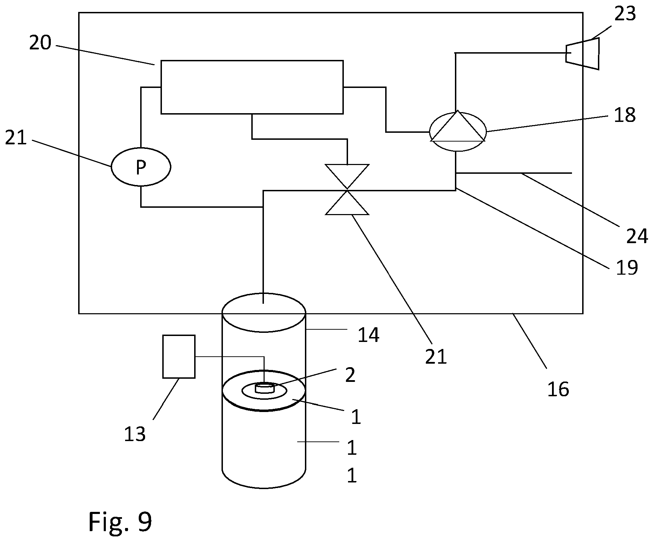

In Fig. 9 ist eine weitere Möglichkeit, der Kopplung schematisch dargestellt. In dieser Ausführungsform erfolgt die Kopplung des Drucksensors 2 and die hydrophobe Membran 1 mittels eines Vakuums. Bei einer solchen Kopplung kann der Drucksensor lösbar an der hydrophoben Membran 1 angebracht werden. Der Drucksensor 2 ist in diesem Fall zumindest in einem Abschnitt seiner Oberfläche nicht für Luft durchlässig. Dazu weist die Behandlungsvorrichtung 16 eine Pumpe 18 und eine Leitung für Luft 19, die mit der zweiten Kammer 14 verbindbar oder verbunden ist, auf. Mit der Pumpe 18 kann Luft aus der zweiten Kammer 14 abgesaugt werden. Optional kann die Behandlungsvorrichtung 16 eine oder mehrere oder alle der folgenden Bauteile aufweisen: eine Steuerung 20, ein Ventil 21, zum Absperren der Leitung für Luft 19 und einer zweiten Druckmesseinrichtung 22, zum Messen des Drucks in der zweiten Kammer 14. Die Steuerung kann mit der Pumpe 18, dem Ventil 21 und der zweiten Druckmesseinrichtung über Signalleitungen verbunden sein. Die Steuerung 20 kann programmiert sein, den Druckwert in der Kammer 14 zu erhalten und, wenn der Druckwert der Kammer 14 oberhalb eines Grenzwerts liegt, die Pumpe 18 zu starten und damit Luft aus der zweiten Kammer 14 abzusaugen. Der Prozessor 13, der das Signal von dem Drucksensor 2 verarbeiten kann, kann in die Steuerung 20 integriert sein, so dass der Prozessor 13, auch programmiert ist, weitere Programmabschnitte der Behandlungsvorrichtung 16 zu verarbeiten, oder der Prozessor 13 kann eine von der Steuerung 20 unabhängiger Prozessor sein und lediglich mit der Steuerung 20 kommunizieren.

Die Pumpe 18 kann eine separat vorgesehen Pumpe zum Absaugen von Luft sein. Die Pumpe 18 kann auch eine Pumpe sein, die dazu genutzt wird, eine Flüssigkeit, insbesondere Dialysat in einer Behandlungsvorrichtung für extrakorporale Blutbehandlung, zu einem Auslass 23 der Behandlungsvorrichtung zu pumpen. Das kann es erlauben, keinen separaten Auslass für das abzuführende Gas vorsehen zu müssen. Dazu kann die Leitung für Luft 19 in eine Leitung für Flüssigkeit 24 münden und die Pumpe 18 stromab des Mündungspunktes angeordnet sein.

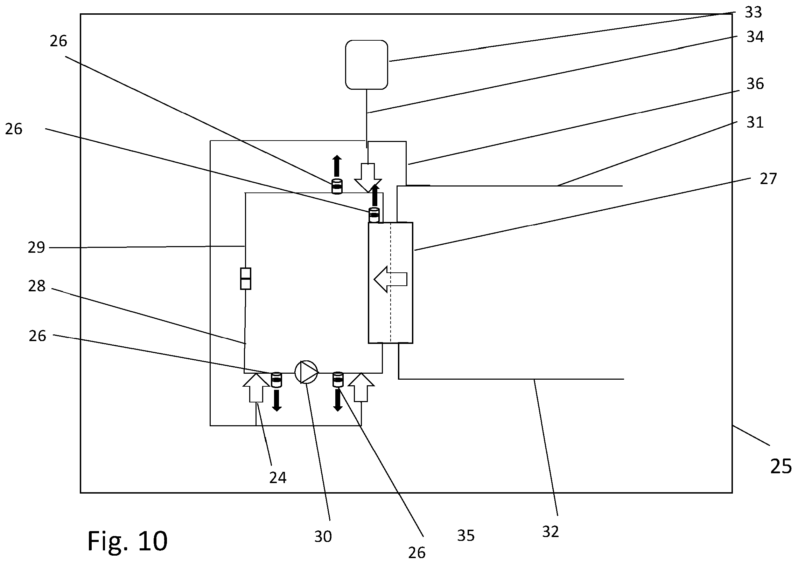

In Fig. 10 sind verschiedene Ausführungsformen eines Behandlungssystems 25 schematisch dargestellt. In dieser Darstellung ist die zuvor beschriebene Druckmesseinrichtung mit der Referenznummer 26 bezeichnet. Die Druckmesseinrichtung kann einmal, zweimal, dreimal oder viermal oder mehr als viermal (nicht dargestellt) an verschiedenen Positionen angeordnet sein. Die Druckmesseinrichtungen 26 können jeweils identisch ausgebildet sein oder entsprechend der oben beschriebenen Ausführungsformen der Druckmesseinrichtungen voneinander abweichend. Die geschlossenen Pfeile (nicht bezeichnet) sind lediglich zur Illustration, wie Gas während eines Füllverfahrens über die Druckmesseinrichtung abgeschieden werden kann. Das Behandlungssystem kann einen oder mehrere Einwegartikel und eine Behandlungsvorrichtung aufweisen. Zum Verständnis des Begriffs Einwegartikel und Behandlungsvorrichtung wird auf die Ausführungen weiter vorne im Text verwiesen. Das Behandlungssystem für eine extrakorporale Blutbehandlung kann folgende Komponenten aufweisen, wobei nachfolgend in Klammer angemerkt ist, ob als Teil eines Einwegartikels oder als Teil der Behandlungsvorrichtung ausgeführt, wobei diese Zuordnung lediglich als optional zu verstehen ist und nicht als zwingend: Einen Dialysator 27 (Einwegartikel), eine arterielle Leitung 28 (Einwegartikel) mit einem Ende verbunden mit einem Eingang des Dialysators 27, eine venöse Leitung 29 (Einwegartikel) mit einem Ende verbunden mit einem Ausgang des Dialysators 27, wobei der Eingang und der Ausgang des Dialysators miteinander fluidisch verbunden sind, eine Blutpumpe 30 (Einwegartikel und Behandlungsvorrichtung), mit der während der Behandlung Blut in Richtung des Dialysators gepumpt wird, wobei die Blutpumpe auf die arterielle Leitung wirken kann. Bei der Blutpumpe 30 kann es sich beispielsweise um eine peristaltische Pumpe handeln, wobei der Aktor der Pumpe Teil der Behandlungsvorrichtung ist und ein Teil der arteriellen Leitung 28 als flexibles Schlauchelement ausgeführt ist, damit der Aktor auf diesen wirken kann. Alternativ kann die Blutpumpe auch eine Membranpumpe sein, wobei die Membran und das Pumpgefäß Teil der arteriellen Leitung 28 ist und die beispielsweise Hydraulik oder Pneumatik oder Mechanik als Aktor, um die Membran zu bewegen, Teil der Behandlungsvorrichtung ist. Alternativ kann die Blutpumpe 30 eine Impellerpumpe, insbesondere eine magnetisch angetriebene Impellerpumpe, sein, bei der der Impeller als Teil der arteriellen Leitung 28 in dieser angeordnet ist, während der Antrieb für den Impeller, insbesondere der magnetische Antrieb, als Teil der Behandlungsvorrichtung ausgebildet ist. Als weitere Elemente können vorgesehen sein: eine Dialysatzufuhrleitung 31, die mit einem Ende mit dem Dialysator 27 verbunden ist, zum Zuführen von Dialysat und/oder Füllflüssigkeit zum Dialysator 27, eine Dialysatabfuhrleitung 32, die mit einem Ende mit dem Dialysator 27 verbunden ist, zum Abführen von Dialysat und/oder Füllflüssigkeit vom Dialysator 27, wobei die Dialysatabfuhrleitung 32 die Leitung für Flüssigkeit 24, wie im Zusammenhang mit Fig. 9 beschrieben, sein kann, ein Gefäß 33, beispielsweise ein Beutel oder ein Kanister, der über eine Leitung 34 mit der arteriellen Leitung 28 und/oder der venösen Leitung 29 verbunden sein kann, wobei der Beutel eine Füllflüssigkeit enthalten kann. Die Dialysatzufuhrleitung 31 kann über eine Leitung 36 mit der arteriellen Leitung 28 und/oder der venösen Leitung 29 verbunden sein. Über die Leitung 36 kann Substituatflüssigkeit während der Behandlung bei einer Hemodiafiltrationsbehandlung oder einer Hemofiltrationsbehandlung zugeführt werden und/oder beim Füllen Dialysat oder Füllflüssigkeit zugeführt werden. Die offenen Pfeile (nicht bezeichnet) sind lediglich zur Illustration, wie Flüssigkeit während eines Füllverfahrens in die arterielle Leitung 28 und/oder der venösen Leitung 29 und/oder den Dialysator 27 überführt werden kann.

Zum Füllen eines Leitungssystems, das die arteriellen Leitung 28, die venöse Leitung 29 und den Dialysator 27 aufweist, können die arterielle Leitung 28 und die venöse Leitung 29 mit Enden, die während einer Behandlung mit dem Patienten verbunden sind, unmittelbar miteinander oder über einen Adapter miteinander verbindbar sein. Der Adapter kann ein T-Stück oder Y Stück sein, dessen dritte Öffnung mit der Dialysatabfuhrleitung 32 verbindbar ist. Die Luft kann zusätzlich zum Abführen über die Druckmesseinrichtung 26 auch über die die dritte Öffnung abführbar sein.

Zum Füllen eines Leitungssystems, das die arteriellen Leitung 28, die venöse Leitung 29 und den Dialysator 27 aufweist, können jeweils ein Ende der arteriellen Leitung 28 und ein Ende der venösen Leitung 28, die jeweils während einer Behandlung mit dem Patienten verbunden sind, einzeln mit der Leitung mit der Dialysatabfuhrleitung 32 verbindbar sein. Für die Verbindungen mit der Leitung mit der Dialysatabfuhrleitung 32 können in der Behandlungsvorrichtung 25 einer oder mehrere Ports, die fluidisch mit der Dialysatabfuhrleitung 32 verbunden sind, vorgesehen sein. Die Luft kann zusätzlich zum Abführen über die Druckmesseinrichtung 26 auch über den oder die Ports abführbar sein.

Zum Füllen eines Leitungssystems, das die arterielle Leitung 28, die venöse Leitung 29 und den Dialysator 27 aufweist, können jeweils ein Ende der arteriellen Leitung 28 und ein Ende der venösen Leitung 29, die jeweils während einer Behandlung mit dem Patienten verbunden sind, einzeln mit dem Gefäß 33 verbindbar sein. In dieser Ausführungsform kann die Blutpumpe ansteuerbar sein, um die Flüssigkeit aus dem Gefäß 33 in das Leitungssystem zu saugen. Die Blutpumpe kann die Flüssigkeit in einem Kreislauf, der das Gefäß 33 mitumfasst, im Kreis pumpen. Die Luft kann zusätzlich zum Abführen über die Druckmesseinrichtung 26 auch durch die semipermeable Membran des Dialysators 27 abführbar sein.

Zum Füllen eines Leitungssystems, das die arterielle Leitung 28, die venöse Leitung 30 und den Dialysator 27 aufweist, kann ein Ende der arteriellen Leitung 28 mit einem Konnektor entlang der venösen Leitung 29 verbindbar sein und ein Ende der venösen Leitung 30 kann mit der Dialysatabfuhrleitung 32 verbindbar sein. Für die Verbindung der venösen Leitung 30 mit der Dialysatabfuhrleitung 32 kann in der Behandlungsvorrichtung 25 ein Port vorgesehen sein, der fluidisch mit der Dialysatabfuhrleitung 32 verbunden ist. Die Luft kann zusätzlich zum Abführen über die Druckmesseinrichtung 26 auch über den oder die Ports abführbar sein.

Zum Füllen eines Leitungssystems, das die arterielle Leitung 28, die venöse Leitung 29 und den Dialysator 27 aufweist, kann ein Ende der arteriellen Leitung 28 mit einem Konnektor entlang der venösen Leitung 30 verbindbar sein und ein Ende der venösen Leitung 29 kann mit dem Gefäß 33 verbindbar sein. In dieser Ausführungsform kann die Blutpumpe ansteuerbar sein, um die Flüssigkeit aus dem Gefäß 33 in die venöse Leitung 30 zu saugen. Die Blutpumpe kann die Flüssigkeit in einem Kreislauf, der das Gefäß 33 nicht mitumfasst, im Kreis pumpen. Die Luft kann zusätzlich zum Abführen über die Druckmesseinrichtung 26 auch durch die semipermeable Membran des Dialysators 27 abführbar sein.

An dieser Stelle wird angemerkt, dass jede einzelne der Verbindungen für das zusätzliche Abführen von Luft verwendbar sein können, Flüssigkeit aus dem Leitungssystem abzuführen und optional dabei das Leitungssystem zu spülen. Das Blutbehandlungssystem kann ferner derart programmiert sein, dass die Verbindungen, die potentiell für das zusätzliche Abführen von Luft nutzbar sind, absperrbar sind, wobei entsprechend ansteuerbare Absperrelemente vorgesehen sein können. Die Luft kann lediglich über die Druckmesseinrichtung abgeführt werden, bis die Flüssigkeit die hydrophobe Membran benetzt und sie luftundurchlässig macht. Anschließend, d.h. mit benetzter hydrophoben Membran, kann die Druckmesseinrichtung eingerichtet sein, den Druck an einem Punkt des Leitungssystems zu messen. Insbesondere kann die Druckmesseinrichtung eingerichtet sein, den Druck in einer nachfolgenden Blutbehandlung zu messen.

Der Begriff "Füllen des Leitungssystems" ist nicht so zu verstehen, dass das Leitungssystem komplett gefüllt werden muss. Es können auch nur Teilabschnitte des Leitungssystems mit dem hier beschriebenen Verfahren und unter Verwendung der hier beschriebenen Druckmesseinrichtung gefüllt werden.

Die Druckmesseinrichtung bzw. die Druckmesseinrichtungen kann bzw. können angeordnet sein
- zwischen einem patientenseitigen Ende der arteriellen Leitung 28 und der Blutpumpe 30 (arterielle Druckmesseinrichtung), und /oder
- zwischen der Blutpumpe 30 und dem Dialysator 27 (Vorfilter Druckmesseinrichtung), und /oder
- an der venösen Leitung 29, insbesondere zwischen dem Dialysator 27 und einer Luftabscheidekammer die an der venösen Leitung 29 angeordnet ist oder an der Luftabscheidekammer oder mit einem separaten Port entlang der venösen Leitung 29 (Nachtfilter Druckmesseinrichtung), und /oder
- an dem Dialysator 27.

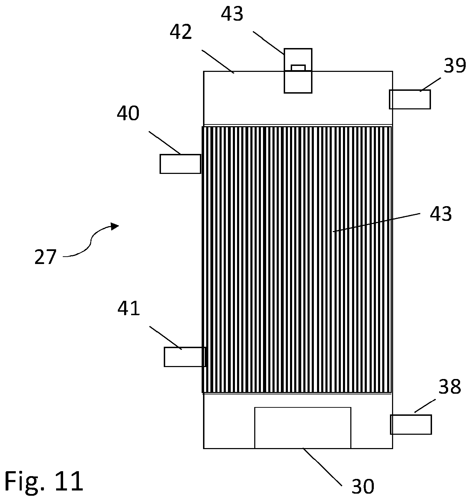

In Fig. 11 ist schematisch eine Ausführungsform dargestellt, bei der die Druckmesseinrichtung in einer Kappe 42 eines Dialysators 27 angeordnet ist. Der Dialysator 27 weist einen Zulauf 38 für Blut (während der Behandlung), einen Ablauf 39 für Blut (während der Behandlung), einen Zulauf 40 für Dialysat (während der Behandlung), einen Ablauf 41 für Dialysat (während der Behandlung) auf. Der Bereich zwischen dem Zulauf für Blut und Ablauf für Blut, die fluidisch miteinander verbunden sind, (Blutkammer) einerseits, und der Bereich zwischen dem Zulauf für Blut und Ablauf für Dialysat, die fluidisch miteinander verbunden sind, (Dialysatkammer) andererseits, sind durch eine semipermeable Membran voneinander getrennt. Bei der semipermeablen Membran kann es sich um ein Bündel von Hohlfasern handeln. Der Ablauf für das Blut ist während des Füllens optional oben angeordnet. Diese Anordnung ist bekannt und wird bei verschiedensten Typen von Dialysatoren eingesetzt. Am oberen Ende kann der Dialysator 27 die Kappe 42 aufweisen mit einer Öffnung 43 und in fluidischer Verbindung mit dem Bereich, in dem während der Behandlung Blut geführt wird. Der Ablauf 39 für Blut kann in bzw. an Kappe 42 angeordnet sein. Über diese Öffnung 43 kann während des Füllens Luft entweichen. An oder als Teil dieser Kappe 42 kann die erfindungsgemäße Druckmesseinrichtung vorgesehen sein. Die Kappe 42 kann kuppelförmig sein und die erfindungsgemäße Druckmesseinrichtung kann an dem obersten Ende der Kuppel angeordnet sein. Dadurch kann Luft in der Kuppel aufsteigen und zu der erfindungsgemäßen Druckmesseinrichtung geleitet werden.

Optional kann im der Kappe 42 gegenüberliegenden Teil des Dialysators 27 die Blutpumpe 30 in den Dialysatorkörper integriert sein, so dass die Blutpumpe 30 und ein Bereich, in dem die Hohlfasern angeordnet sind, ein Bauteil darstellen. Bei der Blutpumpe 30 kann es sich insbesondere um eine Impellerpumpe bzw. einen Impeller einer Impellerpumpe handeln.

Ein Füllverfahren mit dem Blutbehandlungssystem kann folgende Schritte umfassen, wobei diese nicht alle verwirklicht sein müssn:
- Verbinden des Leitungssystems, das die arteriellen Leitung 29, die venöse Leitung 30 und den Dialysator 27 und die erfindungsgemäße Druckmesseinrichtung aufweist, mit einer Flüssigkeitsquelle
- Füllen von Füllflüssigkeit aus einer Füllflüssigkeitsquelle in das Leitungssystem, wobei dieses Füllen umfassen kann
- Pumpen der Füllflüssigkeit aus einer dialysatseitigen Kammer über eine semipermeable Membran in eine blutseitige Kammer des Dialysators 27, wobei das Pumpen durch eine mit der Dialysatzufuhrleitung 31 verbundenen Pumpe erfolgt, und/oder
- Saugen der Füllflüssigkeit aus einer dialysatseitigen Kammer über eine semipermeable Membran in eine blutseitige Kammer des Dialysators 27, wobei das Saugen durch die Blutpumpe 30 erfolgt, und/oder
- Saugen der Flüssigkeit aus einem Gefäß 33, wobei das Saugen durch die Blutpumpe 30 erfolgt, und/oder
- Saugen der Füllflüssigkeit aus einer dialysatseitigen Kammer über eine semipermeable Membran in eine blutseitige Kammer des Dialysators 27 oder dem Gefäß 33, wobei das Saugen durch eine mit der Dialysatabfuhrleitung 32 verbundenen Pumpe erfolgt, und
- Abscheiden der Luft über die Druckmesseinrichtung während des Füllens.

Dabei kann die Luft solange über die Druckmesseinrichtung abgeschieden werden, bis die hydrophobe Membran befeuchtet und luftundurchlässig wird. Ein Ende des Füllens kann erkannt werden und das Pumpen kann beendet werden, wenn die Druckmesseinrichtung erkennt, dass der Druck über einen vorgegebenen Grenzwert steigt. Ein Ende des Füllens kann erkannt werden und das Saugen kann beendet werden, wenn die Druckmesseinrichtung erkennt, dass der Druck unter einen vorgegebenen Grenzwert sinkt.

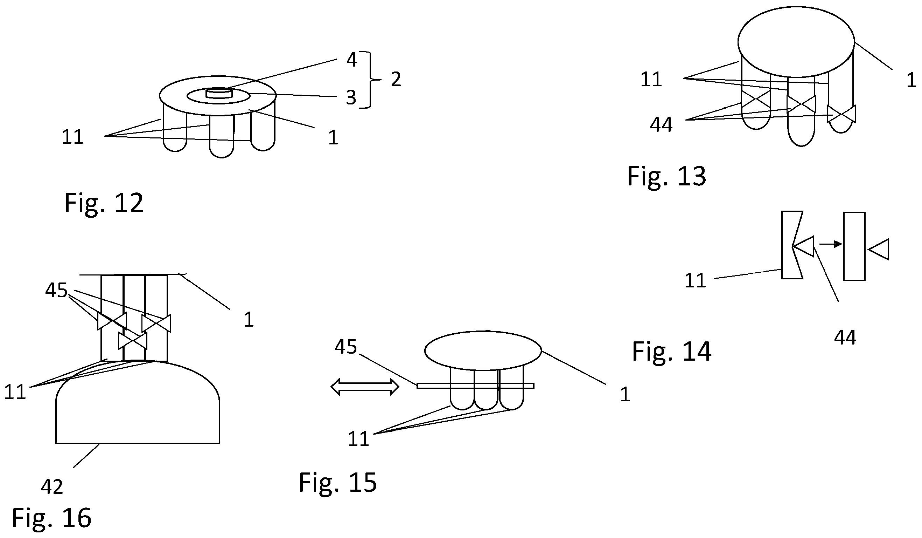

In Fig. 12 ist eine weitere Ausführungsform der Druckmesseinrichtung schematisch dargestellt. Diese unterscheidet sich von den anderen beschriebenen Ausführungsformen dadurch, dass die erste Kammer 11 mehrere Kanäle aufweist, die von der hydrophoben Membran 1 abgeschlossen werden. Die weiteren Merkmale entsprechen den anderen Merkmalen der beschriebenen Ausführungsformen.

In Fig. 13 ist eine Ausführungsform einer Belüftungseinrichtung schematisch dargestellt. Die Belüftungseinrichtung unterscheidet sich von der im Zusammenhang mit Fig. 12 beschriebenen Druckmesseinrichtung dadurch, dass kein Drucksensor 2 vorgesehen ist. Die Belüftungseinrichtung kann ein Verschlussmittel 44 für jeden der der Kanäle der ersten Kammer 11 aufweisen.

In Fig. 14 ist eine Ausführungsform der Kanäle und der Verschlussmittel der Belüftungseinrichtung der im Zusammenhang mit Fig. 13 beschriebenen Belüftungseinrichtung schematisch dargestellt. Die Kanäle können flexible Bereiche, beispielsweise einen Schlauch, aufweisen, die durch das Verschlusselement 44 schließbar, insbesondere zudrückbar, sind.

In Fig. 15 ist eine Ausführungsform der Kanäle und der Verschlussmittel der Belüftungseinrichtung der im Zusammenhang mit Fig. 13 beschriebenen Belüftungseinrichtung schematisch dargestellt. Das Verschlussmittel kann ein Schieber sein, durch den die Kanäle nacheinander durch Verschieben des Schiebers verschließbar sind.

Die Belüftungseinrichtung, beschrieben im Zusammenhang mit den Figuren 11 bis 13, können anstelle der Druckmesseinrichtung an oder als Teil der Kappe 42 eines Dialysators, beschrieben im Zusammenhang mit Fig. 10, vorgesehen sein. Diese Ausführungsform ist schematisch in Fig. 16 dargestellt.

In den im Zusammenhang mit den Figuren 13 bis 16 beschriebenen Ausführungsformen der Belüftungseinrichtung bedeckt die hydrophobe Membran 1 die mehreren Kanäle. In einer Ausführungsform der Belüftungseinrichtung kann die hydrophobe Membran 1 die mehreren Kanäle abdecken und jeden einzelnen der mehreren Kanäle separat abschließen, zum Beispiel mit der Wandung jedes einzelnen Kanals der mehreren Kanäle an der Öffnung des Kanals fest, luft- und fluiddicht verbunden sein, zum Beispiel verklebt, verschweißt, oder gebördelt. In einer Ausführungsform der Belüftungseinrichtung kann jeder der mehreren Kanäle mit einer separaten hydrophoben Membran abdeckt sein und jeder einzelne der mehrere Kanäle separat abgeschlossen, zum Beispiel mit der Wandung an der Öffnung des Kanals fest, luft- und fluiddicht verbunden sein, zum Beispiel verklebt, verschweißt, oder gebördelt.

Ein Füllverfahren für ein Leitungssystems, insbesonders für ein Füllen der arteriellen und/oder der venösen Leitung, kann auch ein Füllen sein, bei der entweder die Druckmesseinrichtung mit der hydrophoben Membran oder eine hydrophobe Membran ohne Druckmesseinrichtung kombiniert mit einer Impellerpumpe als Blutpumpe und optional einer Saugeinrichtung zum Saugen von Flüssigkeit in Richtung der hydrophoben Membran vorgesehen ist. Da die Impellerpumpe, beispielsweise im Gegensatz zu einer peristaltischen Pumpe, keine Luft pumpen kann, muss beim Einsatz einer Impellerpumpe die Flüssigkeit in anderer Art und Weise als durch ein Pumpen bzw. Saugen mittels der Impellerpumpe in das Leitungssystems überführt werden. Die hydrophobe Membran kann dabei keine Membran eines Dialysators sein. Die hydrophobe Membran kann dabei eine Membran eines Dialysators sein. Die hydrophobe Membran kann an einem Leitungsabschnitt des zu füllenden Leitungssystems angeordnet sein.

In einer Ausführung des Füllverfahrens kann die Schwerkraft genutzt werden. Dazu kann eine Flüssigkeitsquelle oberhalb des Leitungssystems angeordnet werden und die Flüssigkeit kann das Leitungssystem mit dem darin angeordneten Impeller füllen.

In einer weiteren Ausführung, die mit der vorhergehend beschriebenen Ausführungsform kombiniert sein kann, kann das Füllverfahren vorsehen, einen Druckgradienten über die hydrophobe Membran zu erzeugen, beispielsweise mittels der Saugeinrichtung, die Teil des Dialysatkreislaufs eines Dialysegeräts sein kann. Insbesondere kann die Saugeinrichtung an einer Dialysatabfuhrleitung des Dialysatkreislaufs angeordnet sein.

Bei dem Füllverfahren kann die zu verdrängende Luft vollständig oder teilweise über die hydrophobe Membran abgeschieden werden. Ist das Leitungssystem soweit gefüllt, dass die hydrophobe Membran benetzt ist, kann keine weitere Luft mehr über die hydrophobe Membran abscheidbar sein.

Das Dialysegerät kann eine Druckmesseinrichtung aufweisen zum Messen des Drucks in der Dialysatabfuhrleitung. Durch eine Steuereinrichtung des Dialysegeräts, die eingerichtet ist, den mittels der weiteren Druckmesseinrichtung ermittelten Druck mit einem Grenzwert zu vergleichen, kann ein Signal zum Stoppen an die Saugeinrichtung übermittelt werden, wenn ein Unterschreiten des Grenzwertes durch die Steuereinheit ermittelt wurde.

Alternativ oder zusätzlich kann eine weitere Druckmesseinrichtung im Dialysegerät vorgesehen sein zum Messen des Drucks in dem Schlauchabschnitt. Durch eine Steuereinrichtung des Dialysegeräts, die eingerichtet ist, den mittels der weiteren Druckmesseinrichtung ermittelten Druck mit einem Grenzwert zu vergleichen, kann ein Signal zum Stoppen an die Saugeinrichtung übermittelt werden, wenn ein Überschreiten des Grenzwertes durch die Steuereinheit ermittelt wurde.

Alternativ oder zusätzlich kann die Steuereinrichtung ein Stoppen der Saugeinrichtung veranlassen, wenn ein vorbestimmtes Flüssigkeitsvolumen von der Saugeinrichtung beim Füllen gefördert wurde.

Das Füllen des Leitungssystems kann in zwei Phasen aufgeteilt sein, einer ersten Phase, in der die Impellerpumpe nicht aktiviert ist, und einer zweiten Phase, in der die Impellerpumpe aktiviert ist. Die erste Phase kann einer Phase entsprechen, in der der Impeller zumindest anfänglich von Luft umgeben ist, und die zweite Phase kann einer Phase entsprechen, in der zumindest zeitweise der Impeller von einer Flüssigkeit umgeben ist. In anderen Worten, beim Füllen kann zunächst die den Impeller umgebende Luft durch Flüssigkeit ersetzt werden und anschließend, wenn Flüssigkeit den Impeller umgibt und folglich eine Pumpwirkung durch den Impeller möglich ist, die Impellerpumpe kann betrieben werden und das Füllen unterstützen.

Ein Wechsel von der ersten zu der zweiten Phase kann dabei volumengesteuert erfolgen. Dabei kann die Steuerung des Dialysegeräts programmiert sein, ein Pumpen der Impellerpumpe zu starten, nachdem ein vorgegebenes Volumen in den Schlauchsatz überführt worden ist.

Alternativ oder zusätzlich kann eine Flüssigkeitserkennungseinrichtung an einer der Schlauchleitungen, insbesondere stromab des Impellers, vorgesehen sein. Die Flüssigkeitserkennungseinrichtung kann beispielsweise optisch oder mittels Ultraschalles oder auf andere Art und Weise erkennen, dass Flüssigkeit anstelle von Luft im Schlauchsatz vorliegt und, weil stromab des Impellers angeordnet, dass der Impeller von Flüssigkeit umgeben sein muss. Die Steuerung kann das Signal der Flüssigkeitserkennungseinrichtung empfangen und programmiert sein, bei Erkennen von Flüssigkeit ein Pumpen der Impellerpumpe zu starten.

Die Impellerpumpe kann eine Pumpe sein, bei der der Impeller magnetisch gelagert wird, d.h. bei Aktivierung wird der Impeller schwebend in der Flüssigkeit gehalten. Da in dieser Situation der Impeller nicht mit den umgebenden Wandungen in mechanischen Kontakt tritt, kann die Aktivierung der Pumpe auch das Risiko einer Beschädigung oder einer Abnutzung des Impellers verringern.

Ein entsprechendes Dialysegerät kann eines, mehrere oder alle folgenden Merkmale aufweisen:
- eine Füllflüssigkeitsquelle, beispielsweise einen Beutel oder einen Kanister oder eine Verbindung zu einem Dialysatzulauf,
- einen Leitungsabschnitt eines Leitungssystems, der mit einem ersten Ende mit der Flüssigkeitsquelle und mit einem zweiten Ende mit einem Dialysator verbunden ist,
- einem Impeller, angeordnet in dem Leitungsabschnitt,
- einen Impellerantrieb, zum Antreiben des Impellers,
- eine entlang des Leitungsabschnitts angeordnete hydrophobe Membran, die das Innere des Leitungsabschnitts nach außen abtrennt,
- eine Saugeinrichtung, die fluidisch mit der hydrophoben Membran verbunden ist und die eingerichtet ist, über die hydrophobe Membran aus dem Leitungsabschnitt abzusaugen,
- eine Druckmesseinrichtung zum Messen des Drucks in einer Leitung zwischen der hydrophoben Membran und der Saugeinrichtung,
- alternativ oder zusätzlich eine weitere Druckmesseinrichtung zum Messen des Drucks in dem Leitungsabschnitt,
- einer Steuereinrichtung zum Starten und Stoppen der Saugeinrichtung und optional zum Erhalten des Messwerts der Druckmesseinrichtung und optional zum Starten des Impellerantriebs, wobei die Steuereinrichtung programmiert sein kann zum Stoppen der Saugeinrichtung, wenn der von der Druckmesseinrichtung übermittelte Druck unter einen vorgegebenen Grenzwert sinkt oder von der

weiteren Druckmesseinrichtung übermittelte Druck über einen vorgegebenen Grenzwert steigt.

## Patentansprüche

1. Eine Druckmesseinrichtung mit,
einer hydrophoben Membran (1), die im trockenen Zustand luftdurchlässig ist und in einem befeuchteten Zustand luftundurchlässig ist, und
einem Drucksensor (2), der mit der hydrophoben Membran (1) in mechanischem Kontakt steht und der eingerichtet ist, einer Bewegung der Membran (1) zu folgen,
einer ersten Kammer (11), die auf einer Vorderseite der hydrophoben Membran (1) angeordnet ist, und
einer zweiten Kammer (14), die auf einer Rückseite der hydrophoben Membran (1) angeordnet ist,
**dadurch gekennzeichnet, dass** der Drucksensor (2) in der zweiten Kammer (14) auf der hydrophoben Membran (1) angeordnet ist.

2. Die Druckmesseinrichtung gemäß Patentanspruch 1 , wobei der Drucksensor eine Druckmesssensorik (4), insbesondere eine Dehnungsmessstreifensensorik oder einen piezoelektrische Sensor, aufweist, und optional eine Trägerschicht (3), auf dem die Druckmesssensorik (4) auf der der hydrophoben Membran (1) abgewandten Seite angeordnet ist, aufweist.

3. Die Druckmesseinrichtung gemäß Patentanspruch 2, wobei die Trägerschicht (3) eine elastische Membran aufweist, deren Randbereich an der ersten Kammer (11) oder der zweiten Kammer (14) vorgespannt befestigt ist, oder wobei die Trägerschicht (3) auf der hydrophoben Membran (1) befestigt ist.

4. Die Druckmesseinrichtung gemäß einem der vorangehenden Patentansprüche, wobei die erste Kammer (11) gemeinsam mit der hydrophoben Membran (1) lösbar an der zweiten Kammer (14) befestigt ist.

5. Die Druckmesseinrichtung gemäß Patentanspruch 4, wobei die erste Kammer (11) gemeinsam mit der hydrophoben Membran (1) und dem Drucksensor (2) lösbar an der zweiten Kammer (14) befestigt ist.

6. Die Druckmesseinrichtung gemäß einem der Patentansprüche 1 bis 4, wobei der Drucksensor (2) ferner eine Kabelleitung (12) aufweist, um ein elektrisches Signal an eine Auswerteeinheit (13) zu übermitteln, wobei die Kabelleitung (12) optional eine Konnektoreinheit aufweist zum Verbinden und/oder Lösen des Drucksensors (2) mit der Auswerteeinheit (13).

7. Die Druckmesseinrichtung gemäß einem der Patentansprüche 1 bis 6, wobei die erste Kammer (11) wenigstens zwei Kanäle aufweist, die jeder an einem Ende von der hydrophoben Membran (1) abgeschlossen werden.

8. Die Druckmesseinrichtung gemäß Patentanspruch 7, wobei mindestens zwei der wenigstens zwei Kanäle einzeln absperrbar sind.

9. Medizinische Funktionseinrichtung wenigstens eine Blutleitung (28, 29) und/oder einen Dialysator (27) aufweisend, zur Verwendung mit einer Druckmesseinrichtung gemäß einem der Patentansprüche 1 bis 8, wobei die erste Kammer (11) und die hydrophobe Membran (1), oder wobei die erste Kammer (11) und die hydrophobe Membran (1) und der Drucksensor (2) Teil der medizinische Funktionseinrichtung sind.

10. Die medizinische Funktionseinrichtung gemäß Patentanspruch 9 mit einem Leitungsabschnitt, der eingerichtet ist zum Einlegen in eine peristaltische Pumpe, oder mit einer Impellerpumpe oder mit einer Membranpumpe, wobei die Druckmesserinrichtung fluidisch vor oder hinter der Pumpe angeordnet ist, und/oder mit einer Luftabscheidekammer, wobei die Druckmesseinrichtung fluidisch vor oder hinter der Luftabscheidekammer angeordnet ist, und/oder mit einem Dialysator (27), wobei die Druckmesseinrichtung an dem Dialysator (27) angeordnet ist.

11. Verfahren zum Messen eines Drucks unter Verwendung einer Druckmesseinrichtung gemäß einem der Patentansprüche 1 bis 8, aufweisend:
- Füllen des Blutschlauchsystems (28, 29) mit einer Flüssigkeit unter Verdrängung von Luft im Blutschlauchsystem (28, 29) durch die hydrophobe Membran (1) hindurch, wobei die hydrophobe Membran (1) von der Flüssigkeit benetzt wird, so dass sie luftundurchlässig wird oder im Bereich von einem der wenigsten zwei Kanäle luftundurchlässig wird, und
- Messen der Veränderung der Lage der Membran (1), insbesondere seiner Form, durch den Drucksensor (2).

## Claims

1. Pressure measuring device having
a hydrophobic diaphragm (1) that is permeable to air in the dry state and airtight in a moistened state, and
a pressure sensor (2) that is in mechanical contact with the hydrophobic diaphragm (1) and configured to follow a movement of said diaphragm (1),
a first chamber (11) arranged on a front side of the hydrophobic diaphragm (1), and
a second chamber (14) arranged on a back side of the hydrophobic diaphragm (1),
**characterized in that** the pressure sensor (2) is arranged in the second chamber (14) on the hydrophobic diaphragm (1).

2. Pressure measuring device according to Claim 1, wherein the pressure sensor comprises a pressure measuring sensor system (4), in particular a strain gauge sensor system or a piezoelectric sensor, and optionally comprises a support layer (3), on which the pressure measuring sensor system (4) is arranged on the side facing away from the hydrophobic diaphragm (1).

3. Pressure measuring device according to Claim 2, wherein the support layer (3) comprises an elastic diaphragm, the edge region of which is secured in prestressed fashion to the first chamber (11) or the second chamber (14), or wherein the support layer (3) is secured to the hydrophobic diaphragm (1).

4. Pressure measuring device according to any of the preceding claims, wherein together with the hydrophobic diaphragm (1), the first chamber (11) is detachably secured to the second chamber (14).

5. Pressure measuring device according to Claim 4, wherein together with the hydrophobic diaphragm (1) and the pressure sensor (2), the first chamber (11) is detachably secured to the second chamber (14).

6. Pressure measuring device according to any of Claims 1 to 4, wherein the pressure sensor (2) further comprises a cable line (12) for transmitting an electrical signal to an evaluation unit (13), wherein the cable line (12) optionally comprises a connector unit for establishing and/or breaking the connection between the pressure sensor (2) and the evaluation unit (13).

7. Pressure measuring device according to any of Claims 1 to 6, wherein the first chamber (11) comprises at least two channels, each of which is closed off at one end by the hydrophobic diaphragm (1).

8. Pressure measuring device according to Claim 7, wherein at least two of the at least two channels can be blocked on an individual basis.

9. Medical functional device comprising at least one blood line (28, 29) and/or a dialyser (27), for use with a pressure measuring device according to any of Claims 1 to 8, wherein the first chamber (11) and the hydrophobic diaphragm (1), or the first chamber (11) and the hydrophobic diaphragm (1) and the pressure sensor (2), are part of the medical functional device.

10. Medical functional device according to Claim 9, having a line section configured for insertion into a peristaltic pump or having an impeller pump or having a diaphragm pump, wherein the pressure measuring device is arranged fluidically upstream or downstream of the pump, and/or having an air separation chamber, wherein the pressure measuring device is arranged fluidically upstream or downstream of the air separation chamber, and/or having a dialyser (27), wherein the pressure measuring device is arranged at the dialyser (27).

11. Method for measuring a pressure using a pressure measuring device according to any of Claims 1 to 8, comprising:
- filling the blood tube system (28, 29) with a fluid while displacing air in the blood tube system (28, 29) through the hydrophobic diaphragm (1), wherein the hydrophobic diaphragm (1) is wetted by the fluid such that it becomes impermeable to air or becomes impermeable to air in the region of one of the least two channels, and
- measuring the change in the position of the diaphragm (1), in particular its shape, by the pressure sensor (2).

## Revendications

1. Dispositif de mesure de pression présentant une membrane (1) hydrophobe, qui est perméable à l'air à l'état sec et qui est imperméable à l'air dans un état humide et
un capteur de pression (2) qui est en contact mécanique avec la membrane (1) hydrophobe et qui est conçu pour suivre un mouvement de la membrane (1),
une première chambre (11) qui est agencée sur une face avant de la membrane (1) hydrophobe et
une deuxième chambre (14) qui est agencée sur une face arrière de la membrane (1) hydrophobe,
**caractérisé en ce que** le capteur de pression (2) est agencé dans la deuxième chambre (14) sur la membrane (1) hydrophobe.

2. Dispositif de mesure de pression selon la revendication 1, le capteur de pression présentant une détection de mesure (4) de pression, en particulier une détection par jauge de contrainte ou un capteur piézoélectrique, et éventuellement une couche support (3) sur laquelle est agencée la détection de mesure (4) de pression sur le côté opposé à la membrane (1) hydrophobe.

3. Dispositif de mesure selon la revendication 2, la couche support (3) présentant une membrane élastique, dont la zone de bord est fixée de manière précontrainte à la première chambre (11) ou à la deuxième chambre (14) ou la couche support (3) étant fixée à la membrane (1) hydrophobe.

4. Dispositif de mesure de pression selon l'une des revendications précédentes, la première chambre (11) étant fixée conjointement avec la membrane (1) hydrophobe de manière amovible à la deuxième chambre (14).

5. Dispositif de mesure de pression selon la revendication 4, la première chambre (11) étant fixée conjointement avec la membrane (1) hydrophobe et le capteur de pression (2) de manière amovible à la deuxième chambre (14).

6. Dispositif de mesure de pression selon l'une des revendications 1 à 4, le capteur de pression (2) présentant en outre un fil de câble (12) destiné à transmettre un signal électrique à une unité d'évaluation (13), le fil de câble (12) présentant éventuellement unité de raccord destinée à relier le capteur de pression (2) à l'unité d'évaluation (13) et/ou à le détacher de celle-ci.

7. Dispositif de mesure de pression selon l'une des revendications 1 à 6, la première chambre (11) présentant au moins deux canaux qui sont fermés respectivement en une extrémité par la membrane (1) hydrophobe.

8. Dispositif de mesure de pression selon la revendication 7, au moins deux desdits au moins deux canaux pouvant être fermés individuellement.

9. Dispositif fonctionnel médical présentant au moins un tuyau (28, 29) pour le sang et/ou un dialyseur (27), destiné à être utilisé avec un dispositif de mesure de pression selon l'une des revendications 1 à 8, la première chambre (11) et la membrane (1) hydrophobe ou la première chambre (11) et la membrane (1) hydrophobe et le capteur de pression (2) faisant partie du dispositif fonctionnel médical.

10. Dispositif fonctionnel médical selon la revendication 9, présentant une section de tuyau qui est conçue pour être placée dans une pompe péristaltique ou présentant une pompe à roue ou présentant une pompe à membrane, le dispositif de mesure de pression étant agencé fluidiquement en amont ou en aval de la pompe et/ou présentant une chambre de séparation d'air, le dispositif de mesure de pression étant agencé fluidiquement en amont ou en aval de la chambre de séparation d'air et/ou un dialyseur (27), le dispositif de mesure de pression étant agencé au niveau du dialyseur (27).

11. Procédé de mesure d'une pression à l'aide d'un dispositif de mesure de pression selon l'une des revendications 1 à 8, présentant :
- le remplissage du système (28, 29) de tuyauterie pour le sang d'un liquide tout en chassant l'air dans le système (28, 29) de tuyauterie pour le sang à travers la membrane (1) hydrophobe, la membrane (1) hydrophobe étant mouillée par le liquide de telle sorte qu'elle devient imperméable à l'air ou qu'elle devient imperméable à l'air dans la zone de l'un desdits au moins deux canaux et
- la mesure de la modification de la position de la membrane (1), en particulier de sa forme, par le capteur de pression (2).
